# EUROPÄISCHE PATENTSCHRIFT

(11) **EP 1 154 845 B1**
(45) Veröffentlichungstag und Bekanntmachung des Hinweises auf die Patenterteilung: **24.03.2004**
(21) Anmeldenummer: 00906248.0
(22) Anmeldetag: 29.01.2000
(51) Int. Cl.: B01F 17/00

(54) **VERWENDUNG VON ETHOXYLIERTEN GLYCERIDGEMISCHEN ALS EMULGATOREN ZUR HERSTELLUNG VON PARAFFINKOHLENWASSERSTOFFEMULSIONEN**
USE OF ETHOXYLATED GLYCERIDE MIXTURES AS EMULSIFIERS FOR PRODUCING PARAFFIN HYDROCARBON EMULSIONS
UTILISATION DE MELANGES DE GLYCERIDES ETHOXYLES COMME EMULSIFIANTS POUR LA PRODUCTION D'EMULSIONS D'HYDROCARBURES PARAFFINIQUES

(30) Priorität: 09.02.1999 DE 19905104
(43) Veröffentlichungstag der Anmeldung: 21.11.2001
(73) Patentinhaber: Cognis Deutschland GmbH & Co. KG, 40589 Düsseldorf (DE)
(72) Erfinder: BIGORRA LLOSAS, Joaquin, E-08203 Sabadell (ES); SANCHEZ, Agustin, E-08921 Santa Coloma de Gramenet (ES); PI SUBIRANA, Rafael, E-08400 Granollers (ES)
(86) Internationale Anmeldenummer: PCT/EP2000/000722
(87) Internationale Veröffentlichungsnummer: WO 2000/047315

(56) Entgegenhaltungen:
- EP-A- 0 217 250

## Beschreibung

Gegenstand der Erfindung ist die Verwendung von ethoxylierten Glyceridgemischen als Emulgatoren zur Herstellung von Paraffinkohlenwasserstoffemulsionen mit Ölgehalten oberhalb von 50 Gew.-%.

Überraschenderweise wurde gefunden, daß der Einsatz von ethoxylierten Glyceridgemischen als Emulgatoren zu Emulsionen führt, die sich gegenüber dem Stand der Technik auch bei hohem Gehalt an Paraffinkohlenwasserstoffen durch besondere Feinteiligkeit sowie Lager- und Temperaturstabilität auszeichnen. So weisen die Emulsionen selbst bei Ölgehalten von etwa 60 Gew.-% noch Tröpfchengrößen unterhalb von 1 µm auf und entmischen sich selbst bei Temperaturlagerung über 3 Wochen nicht.

### Ethoxylierte Glyceridgemische

Bei den ethoxylierten Glyceridgemischen handelt es sich um bekannte Stoffe, die man vorzugsweise herstellt, indem man Triglyceride mit Glycerin umestert und die resultierenden Glyceridgemische gleichzeitig mit Ethylenoxid umsetzt. Gemäß der Erfindung werden ethoxylierte Glyceridgemische einsetzt, die sich von Triglyceriden der Formel (I) ableiten. 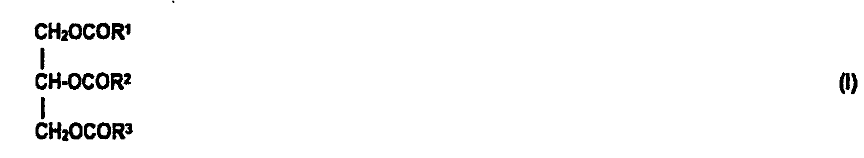 in der R¹CO, R²CO und R³CO unabhängig voneinander für lineare oder verzweigte, gesättigte und/oder ungesättigte Acylreste mit 6 bis 22 und insbesondere 16 bis 18 Kohlenstoffatome stehen. Typische Beispiele hierfür sind die entsprechenden synthetischen Triglyceride auf Basis von Fettsäuren als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden jedoch natürliche Fette und Öle eingesetzt, wie beispielsweise Kokosöl, Palmöl, Palmkernöl, Olivenöl, Rapsöl und insbesondere Rindertalg. Die natürlichen Triglyceride können dabei naturbelassen sein, vorzugsweise weisen sie Jedoch Iodzahlen unterhalb von 20 und insbesondere unterhalb von 5 auf, d.h. sie liegen in gehärtetem oder teilgehärtetem Zustand vor.

### Beschreibung der Erfindung

Gegenstand der Erfindung ist die Verwendung von ethoxylierten Glyceridgemischen als Emulgatoren zur Herstellung von Paraffinkohlenwasserstoffemulsionen.

Überraschenderweise wurde gefunden, daß der Einsatz von ethoxylierten Glyceridgemischen als Emulgatoren zu Emulsionen führt, die sich gegenüber dem Stand der Technik auch bei hohem Gehalt an Paraffinkohlenwasserstoffen durch besondere Feinteiligkeit sowie Lager- und Temperaturstabilität auszeichnen. So weisen die Emulsionen selbst bei Ölgehalten von etwa 60 Gew.-% noch Tröpfchengrößen unterhalb von 1 µm auf und entmischen sich selbst bei Temperaturlagerung über 3 Wochen nicht.

### Ethoxylierte Glyceridgemische

Bei den ethoxylierten Glyceridgemischen handelt es sich um bekannte Stoffe, die man vorzugsweise herstellt, indem man Triglyceride mit Glycerin umestert und die resultierenden Glyceridgemische gleichzeitig mit Ethylenoxid umsetzt. In einer besonders bevorzugten Ausführungsform der Erfindung werden ethoxylierte Glyceridgemische einsetzt, die sich von Triglyceriden der Formel (I) ableiten, 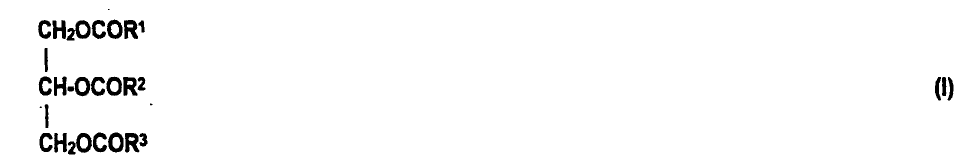 in der R¹CO, R²CO und R³CO unabhängig voneinander für lineare oder verzweigte, gesättigte und/oder ungesättigte Acylreste mit 6 bis 22 und insbesondere 16 bis 18 Kohlenstoffatome stehen. Typische Beispiele hierfür sind die entsprechenden synthetischen Triglyceride auf Basis von Fettsäuren als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden jedoch natürliche Fette und Öle eingesetzt, wie beispielsweise Kokosöl, Palmöl, Palmkemöl, Olivenöl, Rapsöl und insbesondere Rindertalg. Die natürlichen Triglyceride können dabei naturbelassen sein, vorzugsweise weisen sie jedoch Iodzahlen unterhalb von 20 und insbesondere unterhalb von 5 auf, d.h. sie liegen in gehärtetem oder teilgehärtetem Zustand vor.

Zur Herstellung der ethoxylierten Glyceridgemische müssen die Triglyceride in einem ersten Schritt mit Glycerin umgeestert werden. Dies geschieht in an sich bekannter Weise, d.h. als Katalysatoren dienen vorzugsweise Alkalihydroxide oder Alkali-C₁-C₄-Alkoholate, deren Einsatzmenge bezogen auf das Triglycerid üblicherweise im Bereich von 0,1 bis 5 Gew.-% liegt. Als Co-Katalysatoren eignen sich Hypophosphorsäure und deren Alkalisalze, deren Einsatzkonzentration in der Regel bei 10 bis 50 Gew.-% - bezogen auf den Katalysator - beträgt. Die Menge des eingesetzten Glycerins ist in weiten Grenzen variabel und hängt davon ab, welchen Umesterungsgrad man einzustellen wünscht. Üblicherweise beträgt das molare Verhältnis zwischen zugesetztem und im Triglycerid gebundenem Glycerin 1,5 : 1 bis 2,2 : 1 und vorzugsweise 1,8 : 1 bis 2,0 : 1. Aus anwendungstechnischer Sicht hat es sich als vorteilhaft erwiesen, das Einsatzverhältnis so zu wählen, daß bei der Umesterung überwiegend ein Monoester erhalten wird. Typischerweise setzen sich die Partialglyceride wie folgt zusammen:
(a) 50 bis 90, vorzugsweise 60 bis 80 Gew.-% Monoglyceride,
(b) 10 bis 50, vorzugsweise 20 bis 40 Gew.-% Diglyceride und
(c) 0 bis 10, vorzugsweise 3 bis 8 Gew.-% Triglyceride,
mit der Maßgabe, daß sich die Mengenangaben zu 100 Gew.-% ergänzen. In einem zweiten Reaktionsschritt werden die Partialglyceride ethoxyliert. Überwiegend findet die Anlagerung des Ethylenoxids an den primären Hydroxylgruppen der Partialglyceride statt, es können jedoch auch sekundäre Hydroxylgruppen ethoxyliert werden; untergeordnet kommt es sogar zu einer Insertion des Ethylenoxids in die Estercarbonylgruppe. Vorzugsweise werden solche ethoxylierten Glyceridgemische eingesetzt, welche - bezogen den molaren Anteil an Glycerin im Molekül - durchschnittlich 1 bis 50, bevorzugt 5 bis 25 und insbesondere 8 bis 15 Mol Ethylenoxid enthalten. Die ethoxylierten Glyceridgemische können - bezogen auf die Emulsionen - in Mengen von 0,1 bis 10 und insbesondere 0,5 bis 5 Gew.-% eingesetzt werden.

### Fettsäuren

In einer weiteren bevorzugten Ausführungsform der Erfindung hat es sich als vorteilhaft erwiesen, die ethoxylierten Glyceridgemische zusammen mit Fettsäuren einzusetzen, die insbesondere der Formel **(II)** folgen,

**R**^{**4**}**CO-OH** (II)

in der R⁴CO für einen linearen oder verzweigten, gesättigten oder ungesättigten Acylrest mit 6 bis 22 Kohlenstoffatomen steht. Typische Beispiele hierfür sind Capronsäure, Caprylsäure, 2-Ethylhexan-säure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkem- oder Talgfettsäure. Aus anwendungstechnischer Sicht hat es sich als besonders empfehlenswert erwiesen, solche Fettsäuren einzusetzen, deren Acylrest mit dem der ethoxylierten Glyceride korrespondiert. Üblicherweise werden die Fettsäuren in Mengen von 0,5 bis 10 und vorzugsweise 1 bis 2 Gew.-% - bezogen auf die Emulsion - eingesetzt.

### Paraffinemulsionen

Die Paraffinemulsionen werden beispielsweise als Betontrennmittel, Hydrophobiermittel oder Schmierstoffe eingesetzt, es kann sich jedoch auch um Zubereitungen handeln, die als Grundlage zur Herstellung von kosmetischen Zubereitungen dienen. In letzterem Fall können die Emulsionen weitere für diesen Anwendungszweck typische Inhaltsstoffe, wie z.B. milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, UV-Licht-schutzfaktoren, Antioxidantien, anorganische Farbpigmente, Hydrotrope, Konservierungsmittel, Insektenrepellentien, Selbstbräuner, Solubilisatoren, Parfümöle, Farbstoffe und dergleichen enthalten. Ein besonderer Vorteil der vorliegenden Erfindung besteht wie schon eingangs erläutert darin, daß sich nunmehr auch Emulsionen mit ausreichender Feinteiligkeit sowie Lager- und Temperaturstabilität erhalten lassen, die vergleichsweise große Mengen Ölkörper und vorzugsweise 50 bis 70, insbesondere aber etwa 60 Gew.-% Paraffinkohlenwasserstoffe enthalten. Die Auswahl der Paraffinkohlenwasserstoffe, die synonym auch als Mineral- oder Weißöle bezeichnet werden, ist innerhalb weiter Grenzen unkritisch und orientiert sich im wesentlichen daran, daß sie bei Normaltemperatur flüssig vorliegen, wie beispielsweise Paraffinum liquidum (Viskosität 110 bis 230 mPas) oder Paraffinium perliquidum (Viskosität 25 bis 80 mPas).

### Beispiele

**Herstellbeispiel 1.** In einem 2-l-Rührautoklaven wurden 600 g (0,71 mol) gehärteter Rindertalg, 124 g (1,35 mol) Glycerin, 1,2 g Hypophosphorsäure und 2,5 g einer 50 Gew.-%igen wäßrigen Kaliumhydroxidlösung vorgelegt. Der Reaktor wurde verschlossen, aufgeheizt und bei 110°C evakuiert. Bei einer Temperatur von 170°C wurden portionsweise 893 g (20,3 mol) Ethylenoxid aufgegeben, wobei sich ein autogener Druck von etwa 4 bar einstellte. Nach Abschluß der Zugabe wurde die Mischung weitere 30 min gerührt, dann wurde der Reaktor abgekühlt, entspannt und der Katalysator mit Essigsäure neutralisiert. Es wurde ein Gemisch ethoxylierter Glyceride in Form einer leicht gelb gefärbten, niedrigviskosen Flüssigkeit erhalten. Der Ethoxylierungsgrad betrug bezogen auf Glycerin durchschnittlich 10.

**Hersteilbeispiel 2.** Analog Beispiel H1 wurden 600 g gehärteter Rindertalg mit 124 g Glycerin zu einem Partialglyceridgemisch umgeestert und dieses gleichzeitig mit 716 g Ethylenoxid umgesetzt. Es wurde ein Gemisch ethoxylierter Glyceride in Form einer leicht gelb gefärbten Paste erhalten. Der Ethoxylierungsgrad betrug bezogen auf Glycerin durchschnittlich 8.

**Herstellbeispiel 3.** Analog Beispiel H1 wurden 600 g gehärteter Rindertalg mit 124 g Glycerin zu einem Partialglyceridgemisch umgeestert und dieses gleichzeitig mit 1000 g Ethylenoxid umgesetzt. Es wurde ein Gemisch ethoxylierter Glyceride in Form einer leicht gelb gefärbten, niedrigviskosen Flüssigkeit erhalten. Der Ethoxylierungsgrad betrug bezogen auf Glycerin durchschnittlich 14.

**Anwendungstechnische Untersuchungen.** Unter Verwendung unterschiedlicher Emulgatoren wurden verschiedene Paraffinemulsionen hergestellt. Hierzu wurde zunächst eine Phase I, bestehend aus 60 g Paraffin (Festpunkt : 54-58 °C), 1 g Stearinsäure und 1 g Emulgator bei 80 °C vermischt und vorsichtig gerührt (200 Upm). In einem zweiten Reaktor wurden ebenfalls bei 80 °C 0,7 g Diethanolamin in 37,3 g destilliertem Wasser gelöst (Phase 2). Anschließend wurde die Phase 2 portionsweise zur Phase 1 gegeben und die Mischung bei 1000 Upm über einen Zeitraum von 30 min homogenisiert. Schließlich wurde die Emulsion noch einmal in einem Homogenisator bei einem Druck von 126 psi nachbehandelt. Anschließend wurden die Emulsionen über einen Zeitraum von 1 bis 3 Wochen bei 40 °C gelagert und die Feinteiligkeit unter dem Lichtmikroskop beurteilt. Die Kriterien für die Stabilität waren die folgenden: (++) stabil, keine Phasentrennung; (+) geringfügige Trübung; (0) deutliche Trübung; (-) Phasentrennung. Die Ergebnisse sind in Tabelle 1 zusammengefaßt. Die Beispiel 1 bis 3 sind erfindungsgemäß, die Beispiele V1 bis V3 dienen zum Vergleich.

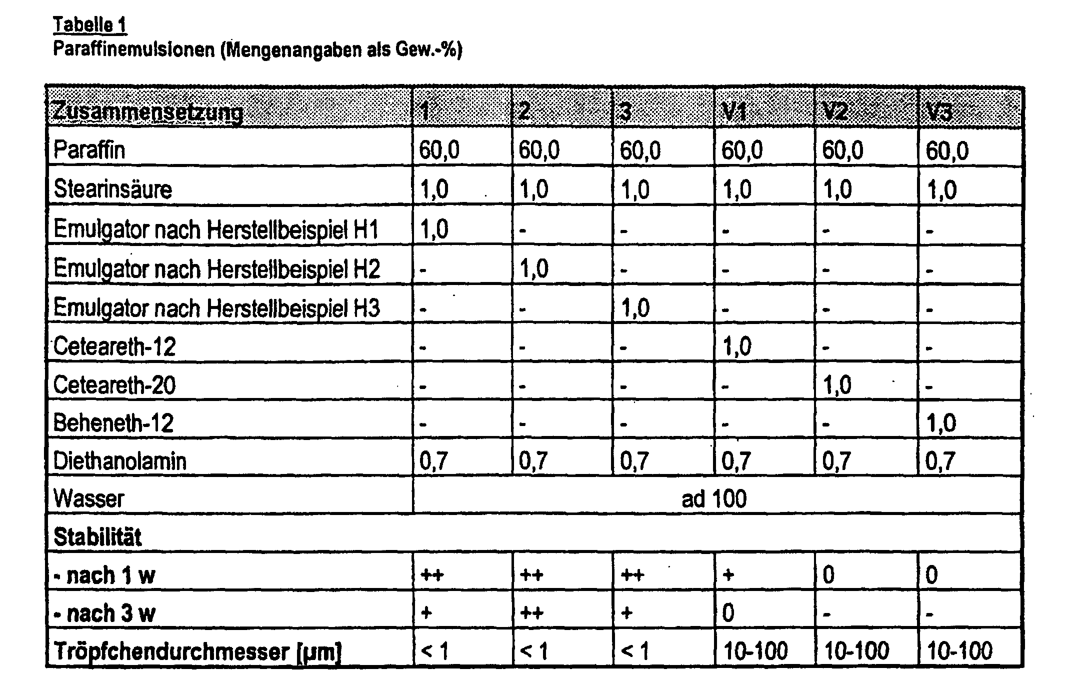

Bei erfindungsgemäßer Verwendung der ethoxylierten Partialglyceride werden Paraffinemulsionen erhalten, die sich bei längerer Temperaturlagerung durch hohe Stabilität und Feinteiligkeit auszeichnen. Mit den Emulgatoren des Stands der Technik werden wesentlich gröbere Emulsionen erhalten, die insbesondere bei Temperaturbelastung nicht ausreichend lagerstabil sind.

## Patentansprüche

1. Verwendung von ethoxylierten Glyceridgemischen als Emulgatoren zur Herstellung von Paraffinkohlenwasserstoffemulsionen mit ölgehalten oberhalb von 50 Gew.-% , und wobei man ethoxylierte Glyceridgemische einsetzt, die sich von Triglyceriden der Formel (I) ableiten, 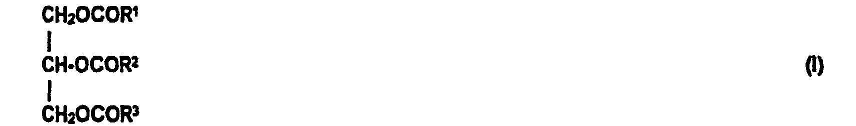 in der R¹CO, R²CO und R³CO unabhängig voneinander für lineare oder verzweigte, gesättigte und/oder ungesättigte Acylreste mit 6 bis 22 Kohlenstoffatome stehen.

2. Verwendung nach Anspruch 1, **dadurch gekennzeichnet, daß** man ethoxylierte Glyceride einsetzt, die man erhält, indem man in an sich bekannter Weise Triglyceride mit Glycerin umestert und die resultierenden Glyceridgemische gleichzeitig mit Ethylenoxid umsetzt.

3. Verwendung nach mindestens einem der Ansprüche 1 bis 3, **dadurch gekennzeichnet, daß** man ethoxylierte Glyceridgemische einsetzt, die sich von Rindertalg ableiten.

4. Verwendung nach mindestens einem der Ansprüche 1 bis 4, **dadurch gekennzeichnet, daß** man ethoxylierte Glyceridgemische einsetzt, die
(a) 50 bis 90 Gew.-% Monoglyceride,
(b) 10 bis 50 Gew.-% Diglyceride und
(c) 0 bis 10 Gew.-% Triglyceride
mit der Maßgabe enthalten, daß sich die Mengenangaben zu 100 Gew.-% ergänzen.

5. Verwendung nach mindestens einem der Ansprüche 1 bis 5, **dadurch gekennzeichnet, daß** man ethoxylierte Glyceridgemische einsetzt, weiche - bezogen den molaren Anteil an Glycerin im Molekül - durchschnittlich 1 bis 50 Mol Ethylenoxid enthalten.

6. Verwendung nach mindestens einem der Ansprüche 1 bis 6, **dadurch gekennzeichnet, daß** man die ethoxylierten Glyceridgemische - bezogen auf die Emulsionen - in Mengen von 0,1 bis 10 Gew.-% einsetzt.

7. Verwendung nach mindestens einem der Ansprüche 1 bis 7, **dadurch gekennzeichnet, daß** man die ethoxylierten Glyceridgemische zusammen mit Fettsäuren der Formel **(II)** einsetzt,
**R**^{**4**}**CO-OH** (II)
in der R⁴CO für einen linearen oder verzweigten, gesättigten oder ungesättigten Acylrest mit 6 bis 22 Kohlenstoffatomen steht.

8. Verwendung nach Anspruch 8, **dadurch gekennzeichnet, daß** man die Fettsäuren in Mengen von 0,5 bis 10 Gew.-% - bezogen auf die Emulsion - einsetzt.

## Claims

1. The use of ethoxylated glyceride mixtures as emulsifiers for the production of paraffin hydrocarbon emulsions with oil contents above 50% by weight, the ethoxylated glyceride mixtures used being derived from triglycerides corresponding to formula (I): 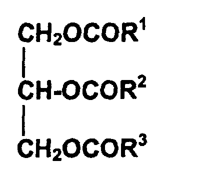 in which R¹CO, R²CO and R³CO independently of one another represent linear or branched, saturated and/or unsaturated acyl groups containing 6 to 22 carbon atoms.

2. The use claimed in claim 1, **characterized in that** the ethoxylated glyceride mixtures used are obtained by transesterifying triglycerides with glycerol and simultaneously reacting the resulting glyceride mixtures with ethylene oxide in known manner.

3. The use claimed in at least one of claims 1 to 2, **characterized in that** ethoxylated glyceride mixtures derived from bovine tallow are used.

4. The use claimed in at least one of claims 1 to 3, **characterized in that** the ethoxylated glyceride mixtures used contain
(a) 50 to 90% by weight monoglycerides,
(b) 10 to 50% by weight diglycerides and
(c) 0 to 10% by weight triglycerides,
with the proviso that the quantities shown add up to 100% by weight.

5. The use claimed in at least one of claims 1 to 4, **characterized in that** the ethoxylated glyceride mixtures used contain on average 1 to 50 mol ethylene oxide, based on the percentage molar content of glycerol in the molecule.

6. The use claimed in at least one of claims 1 to 5, **characterized in that** the ethoxylated glyceride mixtures are used in quantities of 0.1 to 10% by weight, based on the emulsions.

7. The use claimed in at least one of claims 1 to 6, **characterized in that** the ethoxylated glyceride mixtures are used together with fatty acids corresponding to formula **(II):**
**R**^{**4**}**CO-OH** (II)
in which R⁴CO is a linear or branched, saturated or unsaturated acyl group containing 6 to 22 carbon atoms.

8. The use claimed in claim 7, **characterized in that** the fatty acids are used in quantities of 0.5 to 10% by weight, based on the emulsion.

## Revendications

1. Utilisation de mélanges de glycérides éthoxylés en tant qu'agents émulsionnants en vue de la production d'émulsions d'hydrocarbures paraffiniques ayant des teneurs en huile supérieures à 50 % en poids et dans laquelle on met en oeuvre des mélanges de glycérides éthoxylés qui dérivent de triglycérides de formule I 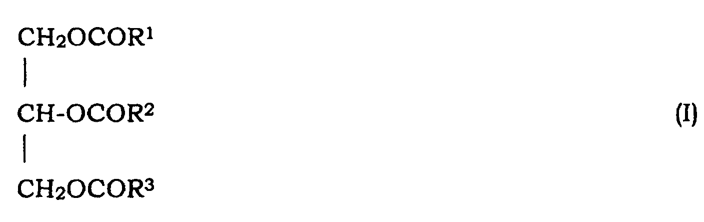 dans laquelle R¹CO, R²CO et R³CO indépendamment les uns des autres représentent des restes acyle linéaires ou ramifiés, saturés et/ou non saturés ayant de 6 à 22 atomes de carbone.

2. Utilisation selon la revendication 1,
**caractérisée en ce qu'**
on met en oeuvre des glycérides éthoxylés que l'on obtient par un procédé dans lequel on transestérifie d'une manière connue en soi des glycérides avec du glycérol et on fait réagir les mélanges de glycérides qui en résultent en même temps avec de l'oxyde d'éthylène.

3. Utilisation selon au moins l'une des revendications 1 à 2,
**caractérisée en ce qu'**
on met en oeuvre des mélanges de glycérides éthoxylés qui dérivent du suif de bovins.

4. Utilisation selon au moins une des revendications 1 à 3,
**caractérisée en ce qu'**
on met en oeuvre des mélanges de glycérides éthoxylés qui renferment
a) de 50 à 90 % en poids de monoglycérides,
b) de 10 à 50 % en poids de diglycérides,
c) de 0 à 10 % en poids de triglycérides avec la précision que les données en quantités se complètent à 100 % en poids.

5. Utilisation selon au moins l'une des revendications 1 à 4,
**caractérisée en ce qu'**
on met en oeuvre des mélanges de glycérides éthoxylés qui renferment - rapporté à la proportion molaire en glycérol dans la molécule ― en moyenne de 1 à 50 mol. d'oxyde d'éthylène.

6. Utilisation selon au moins l'une des revendications 1 à 5,
**caractérisée en ce qu'**
on met en oeuvre les mélanges de glycérides éthoxylés en quantités - rapporté aux émulsions - de 0,1 à 10 % en poids.

7. Utilisation selon au moins l'une des revendications 1 à 6,
**caractérisée en ce qu'**
on met en oeuvre les mélanges de glycérides éthoxylés conjointement à des acides gras de formule (II)
R⁴CO-OH (II)
dans laquelle R⁴CO représente un reste acyle linéaire ou ramifié, saturé ou non saturé ayant de 6 à 22 atomes de carbone.

8. Utilisation selon la revendication 7,
**caractérisée en ce qu'**
on met en oeuvre les acides gras en quantités allant de 0,5 à 10 % en poids - rapporté à l'émulsion.
